# EUROPÄISCHE PATENTSCHRIFT

(11) **EP 0 433 318 B1**
(45) Veröffentlichungstag und Bekanntmachung des Hinweises auf die Patenterteilung: **06.07.1994**
(21) Anmeldenummer: 89909715.8
(22) Anmeldetag: 07.09.1989
(51) Int. Cl.: A61B 17/22, A61B 17/32

(54) **MEDIZINISCHES INSTRUMENT FÜR ATHEREKTOMIE**
SURGICAL INSTRUMENT FOR ATHERECTOMY
INSTRUMENT CHIRURGICAL POUR ATHERIECTOMIE

(30) Priorität: 09.09.1988 DE 3830704
(43) Veröffentlichungstag der Anmeldung: 26.06.1991
(73) Patentinhaber: REDHA, Falah, Dr., 8302 Kloten (CH)
(72) Erfinder: REDHA, Falah, CH-3042 Ortschwaben (CH)
(74) Vertreter: Jeck, Anton, Dipl.-Ing.
(86) Internationale Anmeldenummer: DE8900581
(87) Internationale Veröffentlichungsnummer: WO9002523

(56) Entgegenhaltungen:
- EP-A- 0 117 519
- WO-A-88/00458
- FR-A- 1 585 065
- US-A- 3 837 345

## Beschreibung

Die Erfindung betrifft ein medizinisches Instrument nach dem Oberbegriff des Anspruches 1.

Die Arteriosklerose - chronisch fortschreitende, degenerative Erkrankung vor allem der inneren Arterienwandschicht (Intima) - ist vor allem in den zivilisierten Ländern ein überaus häufiges Krankheitsbild, das bevorzugt in der zweiten Lebenshälfte auftritt und z.B. in etwa 90 % der Fälle Ursache der arteriellen Verschlußkrankheiten ist. Sie steht in der Statistik der Todesursachen an erster Stelle. Durch Eindringen von Blutplättchen in die Zellzwischenräume der innersten Gefäßwandschicht, gefolgt von lipoidhaltigen (fettähnlichen) Ablagerungen, enstehen sog. atheromatöse Veränderungen, die zum Zelluntergang und schließlich zu einer starken Vermehrung der Bindegewebsfasern in dieser Wandschicht führen (Sklerose). Hierdurch verhärtet sich die Arterienwand und büßt ihre natürliche Elastizität ein. Herdförmige Ablagerungen, vor allem von Cholesterinkristallen (Atherome), die auch in den Bereich der mittleren Gefäßwandschicht eindringen, lösen starke, entzündliche Reaktionen und weiteren Gewebszerfall aus.

Schließlich kommt es zu Kalkablagerungen, die Herde brechen auf, und es entstehen Geschwüre, auf denen sich Blutgerinnsel niederschlagen.

Es gibt Fälle, bei denen die Arteriosklerose erfolgversprechend behandelt werden kann. Es besteht die Möglichkeit, Medikamente gegen die Gefäßkrämpfe zu verabreichen, die eine Arteriosklerose oft begleiten, so daß mehr Blut durch die Arterien fließen kann. Bestimmte physiotherapeutische Maßnahmen führen zu einer besseren Ausnutzung des Umgehungskreislaufes. Dazu kommt eine Anzahl chirurgischer Verfahren, die zur Behandlung der Arteriosklerose entwickelt worden sind. Für die Behandlung arteriosklerotischer Veränderungen kommen chirurgische Maßnahmen insbesondere dann in Betracht, wenn ein bestimmtes Organ oder eine bestimmte Körperregion von den Veränderungen betroffen ist. Die Gefäßchirurgie kann insbesondere in folgenden Fällen erfolgreich eingesetzt werden:
a) bei arteriosklerotischen Veränderungen der Halsschlagader, die das Gehirn mit Blut versorgt;
b) bei arteriosklerotischen Veränderungen der Herzkranzgefäße (Koronararterien): Koronarbypass und Einpflanzung der Arteria thoracica interna;
c) bei schweren arteriosklerotisch bedingten Durchblutungsstörungen in den Gliedmaßen;
d) bei Arteriosklerose der Nierenarterie;
e) zur Beseitigung eines Aneurysmas.

Dabei werden regelmäßig bei arteriosklerotischen Veränderungen u.a. folgende Operationsverfahren durchgeführt:
a) die Anlegung eines "Bypass" bei einem Arterienverschluß - d.h., die Verschlußstelle wird mit einem körpereigenen Venenstück oder mit einem entsprechend geformten Kunststoffschlauch als Gefäßersatz überbrückt, so daß der Verschluß umgangen wird;
b) Endarteriektomie;
c) Einsetzen eines "Flickens" nach einer Endarteriektomie;
d) Einsetzen von Gefäß-Prothesen entweder als Ersatz oder zur Umgehung arteriosklerotischer Arterien;
e) Sympathektomie;
f) Behebung von arteriosklerotisch bedingten Gefäßverengungen mittels Ballonkatheter.

Wie bekannt, betreffen die arteriosklerotischen Veränderungen hauptsächlich die innere Auskleidung einer Schlagader, und die äußeren Wandschichten bleiben verhältnismäßig normal. Wenn die sklerotische Innenschicht eines Gefäßes ausgeräumt oder ausgeschält wird, bildet sich in vielen Fällen wieder eine glatte Innenwand. Dies kann z.B. mittels eines Instrumentes erfolgen, wie es in der US-PS 4 765 332 beschrieben ist. Das bekannte Instrument besitzt einen etwa zylinderförmigen Grundkörper mit in Zugrichtung weisenden Schneideabschnitten, die ebenfalls zylinderförmig sind. Die scharfen Kanten der Schneideabschnitte verjüngen sich von innen nach außen, was zur Verletzung auch gesunder Gefäße führen kann.

Ausgehend von dem obigen Stand der Technik liegt der Erfindung die Aufgabe zugrunde, ein medizinisches Instrument zu schaffen, mit dem auch asymmetrische Ablagerungen in den Gefäßwänden problemlos, sicher und ohne Verletzung beseitigt werden können.

Die gestellte Aufgabe wird durch die kennzeichnenden Merkmale des Anspruche 1 gelöst.

Ein solcher Körper kann in das Gefäß mittels eines Katheters eingeführt werden, und zwar bis zur vorgesehenen Stelle, die durch Kontrastmittel lokalisiert werden kann. Der Katheter und der Körper werden so angeordnet, daß die Plaque zwischen dem Körper und dem Katheter angeordnet ist. Dann wird das Zugmittel betätigt und die vorgesehene Stelle wird abgeschabt. Dieser Vorgang kann so oft wiederholt werden, bis eine glatte Innenwand vorhanden ist. Die beim Hobeln entstandenen Späne können entweder abgesaugt oder durch den Körper, der nach dem Eingriff aus der Arterie herausgezogen wird, mitgenommen werden.

Weitere zweckmäßige und vorteilhafte Ausgestaltungen der Erfindung gehen aus den Unteransprüchen hervor.

Eine besonders zweckmäßige Ausgestaltung der Erfindung sieht vor, daß der Körper hohl ist und daß das Zugseil von den Schneidabschnitten umgeben mit der anderen Stirnseite des Hohlkörpers fest verbindbar ist.

Der Vorteil dieser Maßnahme besteht darin, daß alle Späne vom Körper aufgenommen werden können, der nach Benutzung durch einen neuen ersetzt werden kann. Im Rahmen dieses Erfindungsgedankens ist es besonders zweckmäßig, wenn der Hohlkörper im wesentlichen die Form eines glatten Tropfens oder eines Hohlzylinders mit konvexen Stirnseiten besitzt. Durch diese Maßnahme wird vor allem erreicht, daß das Einbringen des Hohlkörpers in die Gefäße erleichtert wird, da die vordere Stirnseite des Hohlkörpers sich verjüngt und somit das Blut besser verdrängen kann.

Beim Betätigen des Zugmittels ist die andere Stirnseite, die dem freien und vom Benutzer betätigten Ende des Zugmittels zugekehrt ist, in Berührung mit der Plaque und den Ablagerungen, wodurch diese beseitigt werden können. Die Konvexität der Stirnseite mit den Schneideabschnitten ist deswegen von Bedeutung, weil dadurch verhindert wird, daß gesundes Gewebe der Arterienwand beschädigt wird. Nur die radial vorstehenden Plaquen werden von den Schneideabschnitten voll erfaßt und beseitigt.

Eine weitere zweckmäßige Ausgestaltung sieht vor, daß der Hohlkörper aus mindestens zwei schalenförmigen und in Zugrichtung sich erstreckenden Teilen besteht, die von einem das Volumen des Hohlkörpers vergrößernden Spannkörper zusammengehalten sind, und daß das mit dem Spannkörper verbindbare Ende des Zugmittels einen Gewindeabschnitt besitzt, der in den Spannkörper einschraubbar ist. Dabei ist es vorteilhaft, wenn der aus einem elastisch verformbaren Werkstoff bestehende Spannkörper eine mittige Bohrung mit Innengewinde besitzt, in die der kegelstumpfförmige Gewindeabschnitt einschraubbar ist.

Alternativ dazu kann vorgesehen sein, daß der aus einem elastisch verformbaren Werkstoff bestehende Spannkörper eine mittige Bohrung mit Innengewinde besitzt und daß das aus der Bohrung herausragende Ende des Gewindeabschnittes eine zum Endabschnitt des Hohlkörpers komplementäre Haube besitzt, die mit dem Endabschnitt in Druckverbindung steht. Durch diese Maßnahme wird ein medizinisches Instrument vorgeschlagen, das nicht nur für Gefäße mit geringem, sondern auch mit großem Durchmesser verwendbar ist.

Der im wesentlichen zylindrische Teil des Hohlkörpers ist gegen die Innenwand der Arterie gedrückt, während die Schneidekanten die Plaque abhobeln können.

Eine weitere zweckmäßige und vorteilhafte Ausgestaltung der Erfindung sieht vor, daß der Hohlkörper axial symmetrisch ist.

Für besonders harte Plaquen können Hohlkörper eingesetzt werden, deren Schneideabschnitte ein zick-zack- oder wellenförmiges Profil aufweisen. Durch diese Sägeform kann die Plaque besonders einfach und schnell beseitigt werden.

Ferner sieht eine Maßnahme der Erfindung vor, daß das im Hohlkörper des Instrumentes angeordnete Ende des Zugseiles mit einem in diesem Hohlkörper bewegbaren Spannstück fest verbunden ist, das bestrebt ist, die Teile des Hohlkörpers voneinander und gegen die Arterienwand zu drücken. Abhängig davon, welche Form das Spannstück besitzt (Kegelstumpf, Pyramidenstumpf, Kugel usw.), wird auf die Innenwand des Hohlkörpers unterschiedlicher Druck ausgeübt, der jedoch so bemessen ist, daß der Hohlkörper, insbesondere seine Schneidekanten, mit den Plaquen in Druckverbindung stehen.

Einige Ausführungsbeispiele der Erfindung sind in der Zeichnung schematisch dargestellt und werden im folgenden näher erläutert. Es zeigen:
- Fig. 1: ein medizinisches Instrument in Seitenansicht und im Querschnitt,
- Fig. 2: ein weiteres medizinisches Instrument in Seitenansicht und Querschnitt,
- Fig. 3: eine dritte Variante des medizinischen Instrumentes in Seitenansicht und Querschnitt,
- Fig. 4: eine weitere Ausführungsform des medizinischen Instrumentes in Seitenansicht und Querschnitt,
- Fig. 5: einen Querschnitt durch eine Arterienwand im gesunden Zustand,
- Fig. 6: einen Querschnitt durch eine Arterienwand bei Arteriosklerose,
- Fig. 7: ein Instrument mit einem durch Druckmedium veränderbaren Spannkörper,
- Fig. 8: das in Fig. 7 dargestellte Instrument mit Sperrkörpern,
- Fig. 9: das in Fig. 7 dargestellte Instrument mit einem anderen Sperrkörper,
- Fig. 10: das in Fig. 7 dargestellte Instrument mit einem Führungsmittel und
- Fig. 11: das in Fig. 7 dargestellte Instrument, dessen Zugmittel einen Führungskörper besitzt.

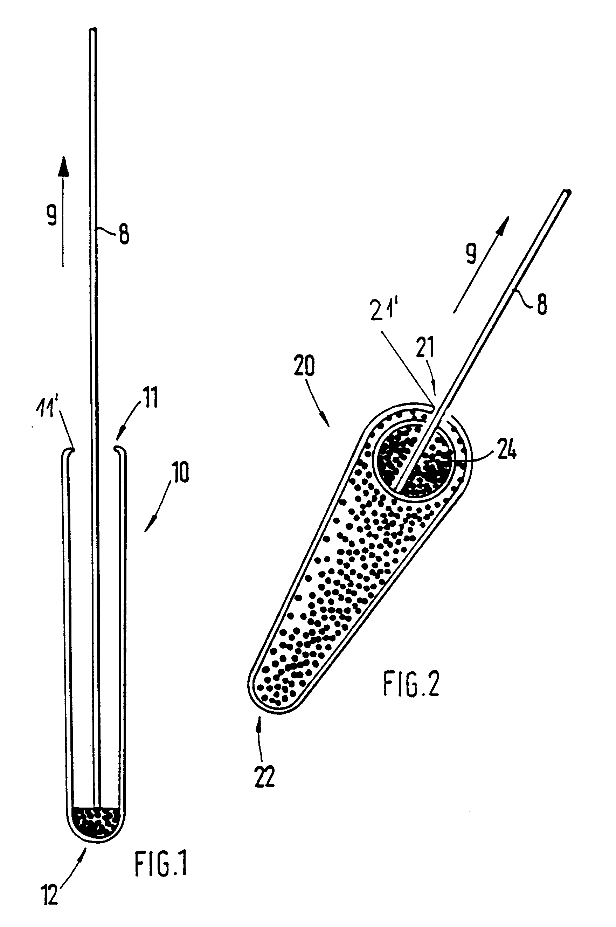

In den Fig. 1 bis 4 sowie Fig. 7 bis 11 sind medizinische Instrumente zur Beseitigung von Ablagerungen in Arterienwänden dargestellt.

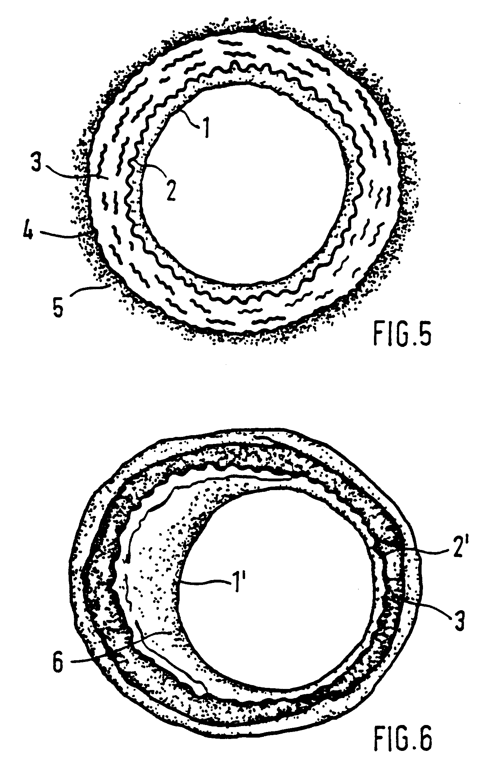

Die Fig. 5 und 6 zeigen im Querschnitt jeweils eine Arterienwand im gesunden Zustand (Fig. 5) und bei Arteriosklerose (Fig. 6). Die gesunde Arterienwand (Fig. 5) besteht aus einer Intima 1, einer Elastica Interna 2, einer Media 3, einer Elastica Externa 4 und einer Adventitia 5.

Bei der Arterienwand bei Arteriosklerose (Fig. 6) ist die Intima 1' bindegewebig verdickt. Das gleiche gilt auch von der Elastica Interna 2'. Zwischen der Elastica Interna und der Intima befindet sich ein metrotischer Intimherd mit Cholesterin 6.

Bei den medizinischen Instrumenten nach den Fig. 1 bis 4 handelt es sich um jeweils einen in die Arterien einbringbaren Körper 10, 20, 30 und 40 mit einem aus den Arterien ausführbaren Zugseil 8, sowie in Zugrichtung 9 weisenden und die Ablagerungen beseitigenden Schneideabschnitten 11, 21, 31 und 41 mit Schneidekanten 11', 21', 31' und 41'.

Man erkennt, daß die Körper 10, 20, 30 und 40 Hohlkörper sind, daß die Schneideabschnitte 11, 21, 31 und 41 im Bereich der der Zugrichtung 9 zugekehrten Stirnseite der Hohlkörper 10, 20, 30 und 40 ausgebildet sind und daß das Zugseil 8, das auch als ein dünnes Röhrchen ausgebildet sein kann, von den Schneideabschnitten 11, 21, 31 und 41 umgeben mit der anderen Stirnseite 12, 22, 32 und 42 des jeweiligen Körpers fest verbindbar ist. Die Hohlkörper 10, 20, 30 und 40 haben im wesentlichen die Form eines glatten Tropfens oder eines Hohlzylinders mit konvexen Stirnseiten. In allen Fällen sind die Schneideabschnitte 11, 21, 31 und 41 zum Zugseil 8 hin gerichtet, so daß es zu keiner Beschädigung der Arterienwand kommen kann. Bei dem medizinischen Instrument nach Fig. 1 ist das Zugseil 8 mit der Stirnseite 12 durch Kleben oder Schweißen verbunden.

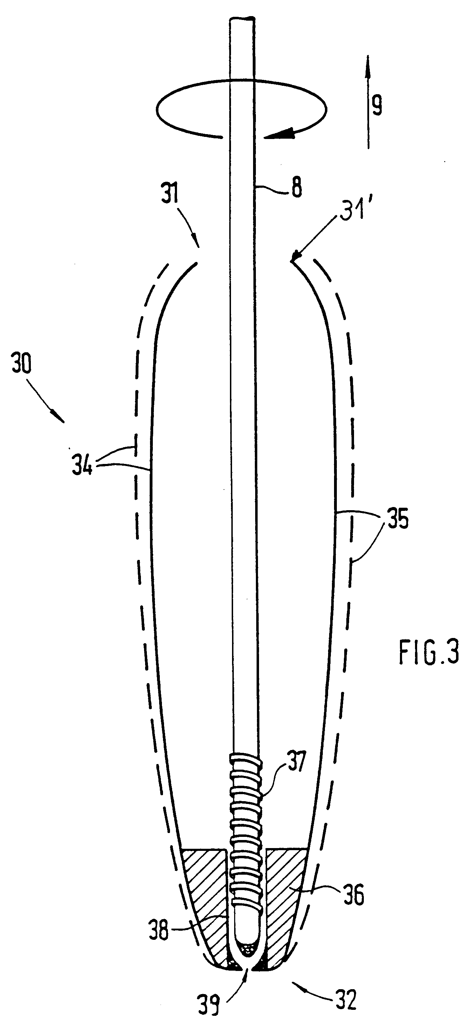

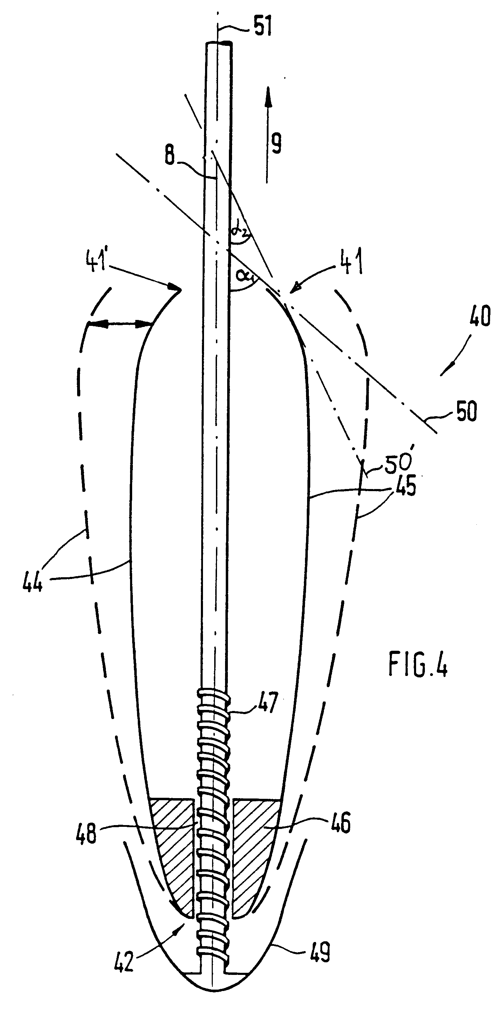

Bei dem medizinischen Instrument nach den Fig. 3 und 4 handelt es sich um jeweils einen Hohlkörper 30 und 40, der aus zwei schalenförmigen und in Zugrichtung 9 sich erstreckenden Teilen 34,35 und 44,45 besteht, die von einem das Volumen des Hohlkörpers 30 bzw. 40 vergrößernden Spannkörper 36 bzw. 46 zusammengehalten sind. Das mit dem Spannkörper 36 bzw. 46 verbindbare Ende des Zugseiles 8 besitzt einen Gewindeabschnitt 37 bzw. 47, der in den Spannkörper 36 bzw. 46 einschraubbar ist.

Bei dem medizinischen Instrument nach Fig. 3 besitzt der aus einem elastisch verformbaren Werkstoff bestehende Spannkörper 36 eine mittige Bohrung 38 mit Innengewinde, in die der kegelstumpfförmige Gewindeabschnitt einschraubbar ist. Das nach außen gerichtete Ende 39 der Bohrung 38 verjüngt sich, so daß beim Einschrauben des Gewindeabschnittes in die Bohrung 38 das Ende 39 erweitert wird, was zur Folge hat, daß das Volumen des Körpers 30 verändert wird.

Die Fig. 4 läßt erkennen, daß der aus einem elastisch verformbaren Werkstoff bestehende Spannkörper 46 eine mittige Bohrung 48 mit Innengewinde besitzt und daß das aus der Bohrung 48 herausragende Ende des Gewindeabschnittes eine zu dem Endabschnitt des Hohlkörpers 40 komplementäre Haube 49 besitzt, die mit dem Endabschnitt 42 in Druckverbindung steht. Die Hohlkörper 10,20,30 und 40 sind axial symmetrisch. Die an den Schneidkanten 41' angrenzenden Tangenten schließen mit der Zentralachse 51 des Hohlkörpers 40 einen Winkel α₁, α₂ ein, der geringer als 90° und größer als 10° ist. Die Schneidkanten 11',21', 31' und 41' können allgemein auch zick-zack-oder wellenförmiges Profil besitzen.

Die Fig. 2 läßt ferner erkennen, daß das im Hohlkörper 20 des Instrumentes angeordnete Ende des Zugseiles 8 mit einem bewegbaren Spannstück 24 fest verbunden ist, das bestrebt ist, die Teile des Hohlkörpers 20 voneinander und gegen die Arterienwand zu drücken. Die Hohlkörper 10, 20, 30 und 40 können aus rostfreiem Stahl oder anderen geeigneten Metallen ausgebildet sein. Es ist jedoch durchaus möglich, auch andere Werkstoffe, wie Kunststoffe, zu benutzen.

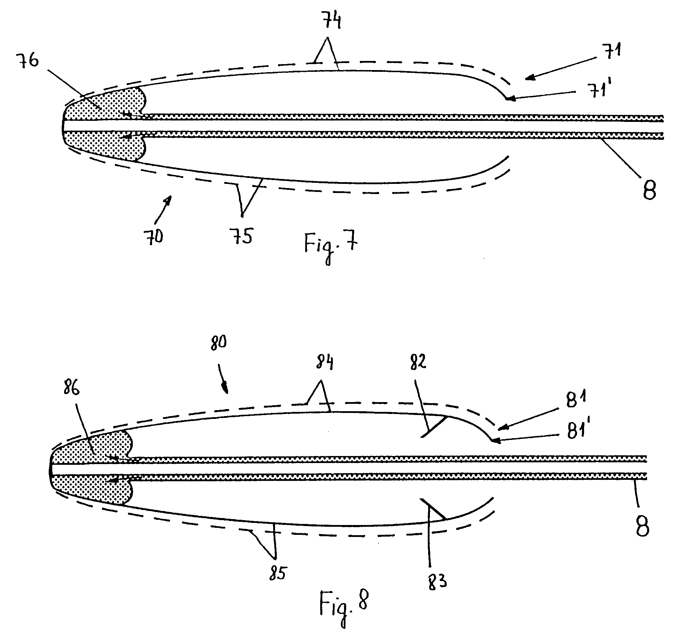

In den Fig. 7 bis 11 sind Körper 70, 80, 90, 100 und 110 mit Schneideabschnitten 71, 81, 91, 101 und 111 dargestellt, an deren Enden scharfe Schneidekanten 71', 81', 91', 101', 111', ausgebildet sind. In den Körpern sind elastisch verformbare und an das Zugmittel 8 angeschlossene Spannkörper 76, 86, 96, 106 und 116 angeordnet. Das Zugmittel kann aus einem Röhrchen oder doppelwandigen Röhrchen ausgebildet sein, um den Spannkörper mit einem Gasmedium oder Flüssigkeit beaufschlagen zu können. In der Ausgangsstellung haben die Spannkörper ein geringeres Volumen, so daß die Teile 74,75; 84,85; 94,95; 104,105 und 114,115 die mit voller Linie dargestellten Positionen einnehmen. Wird nun in die Spannkörper Druckmedium eingeführt, vergrößert sich das Volumen der Spannkörper 76, 86, 96, 106 und 116, so daß die Teile die gestrichelt dargestellten Positionen einnehmen und gegen die Innenwand der Gefäße drücken.

Die Fig. 8 bis 11 lassen erkennen, daß die Innenseite der Teile 84,85; 94,95; 104,105 und 114,115 mit Sperrkörpern 82,83; 92,93; 102,103 und 112,113 versehen sind, die dazu dienen, die Ablagerungen in den Körpern festzuhalten.

In Fig. 9 sind die Sperrkörper 92,93 Teile eines Einsatzes 98, der das Zugmittel 8 im Bereich 99 umgibt und sich an Vorsprüngen 97 stirnseitig abstützt. Die plättchenförmigen Sperrkörper sind nach innen zu geneigt, so daß die Ablagerungen zwischen den Plättchen und dem Zugmittel nach innen, nicht jedoch nach außen gelangen können.

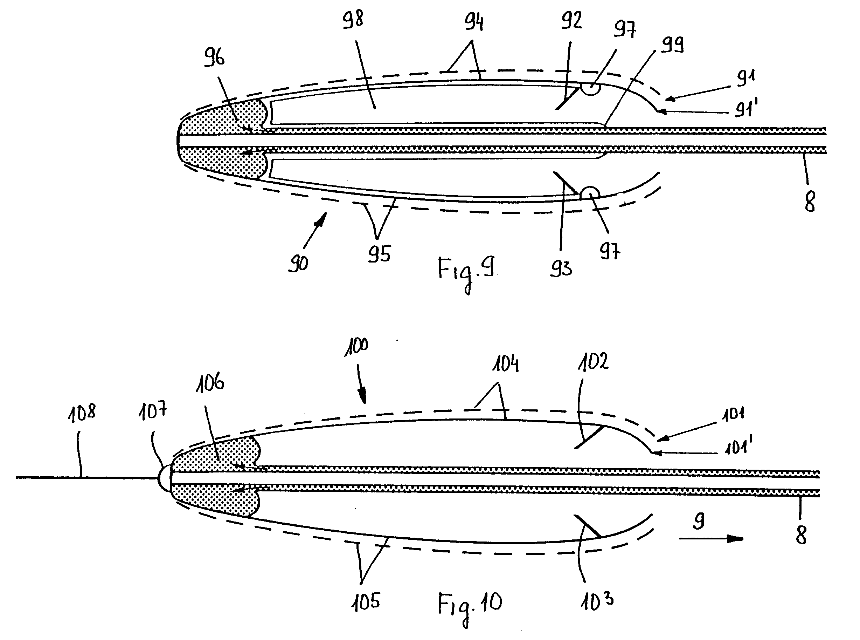

Fig. 10 läßt erkennen, daß das freie Ende des Körpers 100 einen drahtförmigen Führungskörper 118 zur Einführung des Körpers in die Kathetermündung besitzt. Der Führungskörper 108 stützt sich über eine Erweiterung 107 an der Stirnseite des Körpers 100 ab.

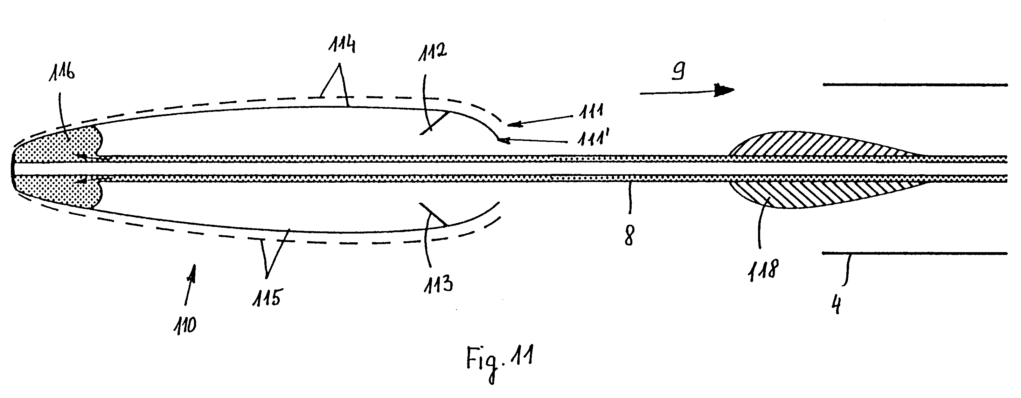

Schließlich zeigt Fig. 11, daß das seilartige Zugmittel 8 ein Führungsmittel 118 besitzt, das tröpfchenförmig ist und sich in Zugrichtung 9 verjüngt.

Die Körper können aus Metall bestehen, wobei deren Außenwand mit einem Isolator beschichtet ist. Lediglich die scharfen Schneidekanten, die mit den Ablagerungen in Berührung kommen, sind ohne Beschichtung. Die Körper stehen mit dem Zugmittel, das ebenfalls aus elektrisch leitendem Material besteht und mit einem Isolator beschichtet ist, elektrisch in Verbindung. Somit kann in die Schneidekanten elektrischer Strom eingeleitet werden, und zwar mit einer Spannung und Intensität, daß die Ablagerungen elektrisch und mechanisch beseitigt werden können.

## Patentansprüche

1. Medizinisches Instrument zur Beseitigung von Ablagerungen in Arterien- und/oder Venenwänden mit einem in die Gefäße einbringbaren, zumindest teilweise hohlen und im Querschnitt etwa kreisrunden Körper (40; Fig. 4) mit einem aus den Gefäßen heraustretenden Zugmittel (8) sowie in Zugrichtung (9) weisenden und die Ablagerungen beseitigenden Schneidabschnitten (41), an deren Ende scharfe Schneidkanten ausgebildet sind,
dadurch gekennzeichnet,
daß die in Zugrichtung (9) weisende Stirnseite des Hohlkörpers (40) konvex gekrümmt ist, so daß der Tangens der Winkel (α₁, α₂), den die Tangenten (50,50') an den äußeren Flächen der Schneideabschnitte (41) mit der Zentralachse (51) des Körpers (40) definieren, von den Schneideabschnitten (41) in Richtung Schneidkante (41') kontinuierlich zunimmt, und
daß die an den Schneidekanten (41) angrenzenden Tangenten (50) die Zentralachse (51) unter einem Winkel (α₁) schneiden, der größer als 10° und kleiner als 90° ist.

2. Medizinisches Instrument nach Anspruch 1,
dadurch gekennzeichnet,
daß der Körper (10,20, 30,40, 70,80, 90,100, 110) hohl ist und
daß das Zugmittel (8) von den Schneideabschnitten (11,21, 31,41) umgeben und mit der anderen Stirnseite (12,22, 32,42) des Hohlkörpers fest verbindbar ist.

3. Medizinisches Instrument nach Anspruch 1 oder 2,
dadurch gekennzeichnet,
daß der Hohlkörper (10,20, 30,40, 70,80, 90,100, 110) im wesentlichen die Form eines glatten Tropfens oder eines Hohlzylinders mit konvexen Stirnseiten besitzt.

4. Medizinisches Instrument nach einem der Ansprüche 1 bis 3, dadurch gekennzeichnet,
daß der Hohlkörper (30,40, 70,80, 90,100, 110) aus mindestens zwei schalenförmigen und in Zugrichtung (9) sich erstreckenden Teilen (34,35; 44,45; 74,75; 84,85; 94,95; 104,105; 114,115) besteht, die von einem das Volumen des Hohlkörpers (30,40, 70,80, 90,100, 110) vergrößernden Spannkörper (36,46) zusammengehalten sind, und
daß das mit dem Spannkörper (36,46) verbindbare Ende des Zugmittels (8) einen Gewindeabschnitt (37,47) besitzt, der in den Spannkörper (36,46) einschraubbar ist.

5. Medizinisches Instrument nach Anspruch 4, dadurch gekennzeichnet,
daß der aus einem elastisch verformbaren Werkstoff bestehende Spannkörper (36) eine mittige Bohrung (38) mit Innengewinde besitzt, in die ein kegelstumpfförmiger Gewindeabschnitt einschraubbar ist.

6. Medizinisches Instrument nach Anspruch 4 oder 5, dadurch gekennzeichnet,
daß der aus einem elastisch verformbaren Werkstoff bestehende Spannkörper (46) eine mittige Bohrung (48) mit Innengewinde besitzt und
daß das aus der Bohrung (48) herausragende Ende des Gewindeabschnittes eine zum Endabschnitt des Hohlkörpers (40) komplementäre Haube (49) besitzt, die mit dem Endabschnitt in Druckverbindung steht.

7. Medizinisches Instrument nach einem der Ansprüche 1 bis 6, dadurch gekennzeichnet,
daß der Hohlkörper (10,20, 30,40, 70,80, 90,100, 110) axialsymmetrisch ist.

8. Medizinisches Instrument nach einem der Ansprüche 1 bis 7, dadurch gekennzeichnet,
daß die Schneideabschnitte ein zick-zack- oder wellenförmiges Profil besitzen.

9. Medizinisches Instrument nach einem der Ansprüche 1 bis 8, dadurch gekennzeichnet,
daß das im Hohlkörper (20) des Instrumentes angeordnete Ende des Zugmittels (8) mit einem in diesem Hohlkörper (20) bewegbaren Spannstück (24) fest verbunden ist, das bestrebt ist, die Teile des Hohlkörpers (20) voneinander und gegen die Arterienwand zu drücken.

10. Medizinisches Instrument nach einem der Ansprüche 4 bis 9, dadurch gekennzeichnet,
daß der aus einem elastisch verformbaren Werkstoff bestehende Spannkörper (76,86, 96,106, 116) an das rohrartig ausgebildete und an Druckmedium anschließbare Zugmittel (8) angeschlossen ist und
daß der Außenumfang des Körpers (70,80, 90,100, 110) durch den Spannkörper (76,86, 96,106, 116) veränderbar ist.

11. Medizinisches Instrument nach einem der Ansprüche 1 bis 10, dadurch gekennzeichnet,
daß innerhalb des hülsenartigen Körpers (80,90, 100,110) elastisch verformbare und schräg nach innen abfallende Sperrkörper (82,83; 92,93; 102,103; 112,113) zum Festhalten der innerhalb des Körpers (80,90, 100,110) angeordneten Ablagerungen ausgebildet sind.

12. Medizinisches Instrument nach Anspruch 11,
dadurch gekennzeichnet,
daß die Sperrkörper (82,83; 92,93; 102,103; 112,113) als angeformte Blättchenfedern ausgebildet sind.

13. Medizinisches Instrument nach Anspruch 11 oder 12,
dadurch gekennzeichnet,
daß die Sperrkörper (92,93) ein Teil eines in den Körper (90) einbringbaren Einsatzes (98) sind, der sich an Festhaltemitteln (97) des Körpers (90) abstützt.

14. Medizinisches Instrument nach einem der Ansprüche 1 bis 13, dadurch gekennzeichnet,
daß das von den Schneidekanten (101') abgekehrte Ende des Körpers (100) mit einem Führungsmittel (108) zur Überwindung der Gefäßstenose versehen ist.

15. Medizinisches Instrument nach einem der Ansprüche 1 bis 14, dadurch gekennzeichnet,
daß das Zugmittel (8) mit einem vor dem Schneideabschnitt (111) angeordneten Führungskörper (108) zur Einführung des Körpers (100) in die Kathetermündung versehen ist.

16. Medizinisches Instrument nach einem der Ansprüche 1 bis 15, dadurch gekennzeichnet,
daß das Führungsmittel (118) die Form eines Tröpfchens besitzt, wobei sein Außendurchmesser geringer ist als der Außendurchmesser des Körpers (110).

17. Medizinisches Instrument nach einem der Ansprüche 1 bis 16, dadurch gekennzeichnet,
daß die Körper (80,90, 100,110) aus elektrisch leitendem Werkstoff bestehen und bis auf die scharfen Schneidekanten (81',91'; 101',111') mit einem Isolator beschichtet sind und
daß das Zugmittel (8) aus einem elektrisch leitenden Werkstoff besteht, mit den Körpern elektrisch verbunden und mit einem Isolator beschichtet ist.

## Claims

1. Medical instrument for removing deposits in artery and/or vein walls, having a body (40; Fig. 4), which is insertable into the vessels, is at least partially hollow and has a roughly circular cross-section, a drawing means (8) which emerges from the vessels, and cutting portions (41), which are orientated in the drawing direction (9) and remove the deposits, sharp cutting edges being provided at the ends of said portions, characterised in that the end face of the hollow body (40) orientated in the drawing direction (9) is convexly curved so that the tangent of the angles (α₁, α₂), which is defined by the tangents (50,50') at the outer faces of the cutting portions (41) with the central axis (51) of the body (40), increases continuously from the cutting portions (41) in the direction towards the cutting edge (41'), and in that the tangents (50), adjacent the cutting edges (41'), intersect the central axis (51) at an angle (α₁), which is greater than 10° and smaller than 90°.

2. Medical instrument according to claim 1, characterised in that the body (10,20, 30,40, 70,80, 90,100, 110) is hollow, and in that the drawing means (8) is surrounded by the cutting portions (11,21, 31,41) and is securedly connectable to the other end face (12,22, 32,42) of the hollow body.

3. Medical instrument according to claim 1 or 2, characterised in that the hollow body (10,20, 30,40, 70,80, 90,100, 110) has substantially the configuration of a smooth drop or a hollow cylinder with convex end faces.

4. Medical instrument according to one of claims 1 to 3, characterised in that the hollow body (30,40, 70,80, 90,100, 110) comprises at least two dish-shaped parts (34,35; 44,45; 74,75; 84,85; 94,95; 104,105; 114,115), which are held together by a clamping member (36,46), which increases the volume of the hollow body (30,40, 70,80, 90,100, 110), and in that the end of the drawing means (8), which is connectable to the clamping member (36,46), has a threaded portion (37,47) which is screw-connectable into the clamping member (36,46).

5. Medical instrument according to claim 4, characterised in that the clamping member (36), which is formed from a resiliently deformable material, has a central bore (38) provided with an internal thread, a frustoconical threaded portion being screw-connectable into said bore.

6. Medical instrument according to claim 4 or 5, characterised in that the clamping member (46), which is formed from a resiliently deformable material, has a central bore (48) provided with an internal thread, and in that the end of the threaded portion protruding from the bore (48) has a cap (49), which is complementary to the end portion of the hollow body (40) and is connected to the end portion in a push-fit manner.

7. Medical instrument according to one of claims 1 to 6, characterised in that the hollow body (10,20, 30,40, 70,80, 90,100, 110) is axially symmetrical.

8. Medical instrument according to one of claims 1 to 7, characterised in that the cutting portions have a zigzag or undulatory profile.

9. Medical instrument according to one of claims 1 to 8, characterised in that the end of the drawing means (8) disposed in the hollow body (20) of the instrument is securedly connected to a clamping piece (24), which is displaceable in this hollow body and seeks to urge the parts of the hollow body (20) apart from one another and towards the artery wall.

10. Medical instrument according to one of claims 4 to 9, characterised in that the clamping member (76,86, 96,106, 116), which is formed from a resiliently deformable material, communicates with the tubular drawing means which is communicatable with pressure medium, and in that the outer periphery of the body (70,80, 90,100, 110) is changeable by the clamping member (76,86, 96,106, 116).

11. Medical instrument according to one of claims 1 to 10, characterised in that resiliently deformable and inclinedly inwardly descending blocking members (82,83; 92,93; 102,103; 112,113) are provided internally of the sleeve-like body (80,90, 100,110) for securedly retaining the deposits disposed internally of the body (80,90, 100,110).

12. Medical instrument according to claim 11, characterised in that the blocking members (82,83; 92,93; 102,103; 112,113) are provided as integrally moulded small plate springs.

13. Medical instrument according to claim 11 or 12, characterised in that the blocking members (92,93) form part of an insert (98), which is insertable into the body (90) and is supported on retaining means (97) for securedly retaining the body (90).

14. Medical instrument according to one of claims 1 to 13, characterised in that the end of the body (100) remote from the cutting edges (101') is provided with a guide means (108) for overcoming the vessel stenosis.

15. Medical instrument according to one of claims 1 to 14, characterised in that the drawing means (8) is provided with a guide means (108), which is disposed in front of the cutting portion (111) for the insertion of the body (100) into the catheter mouth.

16. Medical instrument according to one of claims 1 to 15, characterised in that the guide means (118) has the configuration of a droplet, its external diameter being smaller than the external diameter of the body (110).

17. Medical instrument according to one of claims 1 to 16, characterised in that the bodies (80,90, 100,110) are formed from an electrically conductive material and are coated with an insulator down to the sharp cutting edges (81',91'; 101',111'), and in that the drawing means (8) is formed from an electrically conductive material, is electrically connected to the bodies and is coated with an insulator.

## Revendications

1. Instrument chirurgical pour l'élimination de dépôts sur les parois d'artères et/ou de veines avec un corps au moins partiellement creux et à section transversale sensiblement circulaire (40; Fig. 4) pouvant être introduit dans les vaisseaux, corps muni d'un moyen de traction (8) émergeant du vaisseau et avec des tronçons de coupe (41) orientés dans la direction de traction (9) et éliminant les dépôts, tronçons aux extrémités desquels sont formées des arêtes de coupe vives,
caractérisé
par le fait que la face frontale du corps creux (40) orientée dans la direction de traction (9) présente une courbure convexe, de telle sorte que la valeur des angles (α₁, α₂) que définissent avec l'axe central (51) du corps (40) les tangentes (50, 50') sur les faces extérieures des tronçons de coupe (41) augmente progressivement au fur et à mesure que les tangentes se rapprochent de l'arête de coupe (41'), et
par le fait que les tangentes (50) sur les faces extérieures proches de l'arête de coupe (41) coupent l'axe central (51) sous un angle ( α₁ ) qui est supérieur à 10° et inférieur à 90°.

2. Instrument chirurgical suivant la revendication 1,
caractérisé par le fait
que le corps (10,20, 30,40, 70,80, 90,100, 110) est creux et que le moyen de traction (8) est entouré par les tronçons de coupe (11,21, 31,41) et peut être relié solidement à l'autre face frontale (12,22, 32,42) du corps creux.

3. Instrument chirurgical suivant la revendication 1 ou la revendication 2,
caractérisé par le fait
que le corps creux (10,20, 30,40, 70,80, 90,100, 110) a en substance la forme d'une goutte lisse ou d'un cylindre creux à faces frontales convexes.

4. Instrument chirurgical suivant l'une quelconque des revendications de 1 à 3,
caractérisé par le fait
que le corps creux (30,40, 70,80, 90,100, 110) est composé d'au moins deux parties (34,35; 44,45; 74,75; 84,85; 94,95; 104,105; 114,115) en forme de coquille et orientées dans la direction de traction (9), parties qui sont réunies par un élément tendeur (36,46) qui fait augmenter le volume du corps creux (30,40, 70,80, 90,100, 110), et
que l'extrémité du moyen de traction (8) qui peut être reliée à l'élément tendeur (36,46) présente une portion filetée (37,47) qui peut être vissée dans l'élément tendeur (36,46).

5. Instrument chirurgical suivant la revendication 4,
caractérisé par le fait
que l'élément tendeur (36) fabriqué en un matériau déformable élastiquement présente une forure centrale (38) à filetage intérieur, dans laquelle peut être vissé un tronçon fileté de forme tronconique.

6. Instrument chirurgical suivant la revendication 4 ou la revendication 5,
caractérisé par le fait
que l'élément tendeur (46) constitué par un matériau déformable élastiquement présente une forure centrale (48) à filetage intérieur et
que l'extrémité du tronçon fileté émergeant de la forure (48) possède un capuchon (49) complémentaire au troncon terminal du corps creux (40), capuchon qui est en liaison de pression avec le troncon terminal.

7. Instrument chirurgical suivant l'une quelconque des revendications de 1 à 6,
caractérisé par le fait
que le corps creux (10,20, 30,40, 70,80, 90,100, 110) est à symétrie axiale.

8. Instrument chirurgical suivant l'une quelconque des revendications de 1 à 7,
caractérisé par le fait
que les tronçons de coupe ont un profil en zigzag ou ondulé.

9. Instrument chirurgical suivant l'une quelconque des revendications de 1 à 8,
caractérisé par le fait
que l'extrémité du moyen de traction (8) disposée dans le corps creux (20) de l'instrument est reliée solidement à un élément tendeur (24) mobile dans ce corps creux, élément tendeur qui tend à éloigner l'une de l'autre les parties du corps creux (20) et à les appliquer contre la paroi de l'artère.

10. Instrument chirurgical suivant l'une quelconque des revendications de 4 à 9,
caractérisé par le fait
que l'élément tendeur (76,86, 96,106, 116) constitué par un matériau déformable élastiquement est raccordé au moyen de traction (8) de forme tubulaire et pouvant être raccordé à un fluide de pression et
que le contour extérieur du corps (70,80, 90,100, 110) peut être modifié par l'élément tendeur (76,86, 96,106, 116).

11. Instrument chirurgical suivant l'une quelconque des revendications de 1 à 10,
caractérisé par le fait
qu'à l'intérieur du corps en forme de douille (80,90, 100,110) sont formés des éléments de blocage (82,83; 92,93; 102,103; 112,113) déformables élastiquement et en pente descendante oblique vers l'intérieur pour la retenue des dépôts accumulés à l'intérieur du corps (80,90, 100,110).

12. Instrument chirurgical suivant la revendication 11
caractérisé par le fait
que les éléments de blocage (82,83; 92,93; 102,103; 112,113) ont la forme de lames de ressort intégrées.

13. Instrument chirurgical suivant la revendication 11 ou la revendication 12,
caractérisé par le fait
que les éléments de blocage (92,93) font partie d'une pièce (98) pouvant être placée dans le corps (90) , pièce qui s'appuie sur des moyens de retenue (97) du corps (90).

14. Instrument chirurgical suivant l'une quelconque des revendications de 1 à 13,
caractérisé par le fait
que l'extrémité du corps (100) qui est éloignée des arêtes de coupe(101') est munie d'un moyen de guidage (108) destiné à vaincre la sténose des vaisseaux.

15. Instrument chirurgical suivant l'une quelconque des revendications de 1 à 14,
caractérisé par le fait
que le moyen de traction (8) est muni d'un élément de guidage (108) disposé devant le tronçon de coupe (111) pour l'introduction du corps dans l'orifice du cathéter.

16. Instrument chirurgical suivant l'une quelconque des revendications de 1 à 15,
caractérisé par le fait
que le moyen de guidage (118) a la forme d'une gouttelette, dont le diamètre extérieur est inférieur au diamètre extérieur du corps (110).

17. Instrument chirurgical suivant l'une quelconque des revendications de 1 à 16,
caractérisé par le fait
que le les corps (80,90, 100,110) sont réalisés en un matériau conducteur électrique et sauf pour ce qui concerne les arêtes de coupe vives (81',91', 101',111') sont revêtus d'un isolant et
que le moyen de traction (8) est réalisé en un matériau conducteur électrique, est connecté électriquement aux corps et est revêtu d'un isolant.
